# EUROPEAN PATENT APPLICATION

(11) **EP 3 121 259 A1**
(43) Date of publication of application: **25.01.2017**
(21) Application number: 16179936.6
(22) Date of filing: 18.07.2016
(51) Int. Cl.: C12M 1/00, C12M 1/26, C12M 1/34

(54) **CELL CULTURING APPARATUS**

(30) Priority: 23.07.2015 JP 2015145830; 27.05.2016 JP 2016105714
(71) Applicant: Olympus Corporation, Tokyo 192-8507 (JP)
(72) Inventor: Kimura, Hiroyuki, Hachioji-shi, Tokyo 192-8507 (JP); Minami, Tatsuya, Hachioji-shi, Tokyo 192-8507 (JP); Makara, Yasunori, Hachioji-shi, Tokyo 192-8507 (JP)
(74) Representative: Schicker, Silvia

(57) **Abstract**

A cell culturing apparatus (100) according to the present invention includes a culture-medium retaining means (3) that retains a culture medium (A) for culturing a cell, a culture bag (2) provided with a feed port (2a) and a discharge port (2b), a culture-medium feeding means (4) that connects the culture-medium retaining means (3) and the feed port (2a) and that feeds the culture medium (A) from the culture-medium retaining means (3) to the culture bag (2), a negative-pressure supplying means (6) that supplies negative pressure to the discharge port (2b), and a waste retaining means (5) that retains the culture medium (A) from the discharge port (2b).

## Description

### {Technical Field}

The present invention relates to cell culturing apparatuses that are capable of automatically replacing culture media within culture bags (cell culture bags).

### {Background Art}

With recent developments in stem-cell research and regenerative medicine, there are demands for preparing a large number of cells for clinical use. For preparing cells for clinical use, it is demanded that the procedure be performed in an environment that complies with strict standards. Therefore, an enormous amount of labor and cost is necessary, such as operators changing into disposable work outfits before entering a work space. In addition, the procedure performed by the operator creates an opportunity for contamination of the culturing system. Therefore, it is demanded that the number of times the operator enters the work space and performs the procedure be reduced as much as possible and that some procedures be performed automatically without involving human work, if possible.

Although a culture medium (cell culture solution) needs to be replaced periodically for culturing cells, such culture-medium replacement involves the risk of contamination of the culturing system and therefore should be performed automatically without human intervention as much as possible. A known system for automatically replacing a culture medium uses a conveying robot to move a culture container between an incubator and a culture-medium replacing robot (for example, see Patent Literature 1).

### {Citation List}

### {Patent Literature}

{PTL 1} Japanese Unexamined Patent Application, Publication No. 2002-262856

### {Summary of Invention}

### {Technical Problem}

Since strict standards are set for preparing cells for clinical use, and the risks of contamination of the culturing system need to be eliminated as much as possible, it is advantageous to automate the culture-medium replacing process. However, in the automatic culture-medium replacing system in Patent Literature 1, the system is extremely complex due to the use of, for example, the conveying robot, thus increasing the risks of system errors. Moreover, since the culture container is inserted into and removed from the incubator, the cells within the culture container experience stress caused by temperature changes. If the system is set within the incubator to avoid this, the high-humidity environment within the incubator may lead to the risk of trouble in mechanical or electrical structures. Therefore, it is preferable that the system components within the incubator have configurations that are as simple as possible.

The present invention has been made in view of the circumstances described above, and an object thereof is to provide a cell culturing apparatus with which a culture medium within a culture bag (cell culture bag) in a cell culture space can be simply and automatically replaced and in which the risks of system trouble can be reduced by simplifying the configuration of the apparatus.

### {Solution to Problem}

In order to achieve the aforementioned object, the present invention provides the following solutions.

An aspect of the present invention provides a cell culturing apparatus including a culture-medium retaining means that retains a culture medium for culturing a cell, a culture bag having a feed port to which the.culture medium is fed and a discharge port through which the culture medium is discharged from inside the culture bag, a culture-medium feeding means that connects the culture-medium retaining means and the feed port of the culture bag and that feeds the culture medium discharged from the culture-medium retaining means to the culture bag, a negative-pressure supplying means that supplies negative pressure to the discharge port of the culture bag, and a waste retaining means that retains the culture medium from the discharge port of the culture bag.

According to this aspect, the culture medium in the culture bag can be replaced by using a simple configuration.

In the above aspect, the culture-medium feeding means may include a feed-rate adjusting means that adjusts a rate at which the culture medium is fed to the culture bag. Accordingly, the feed rate of the culture medium toward the culture bag can be arbitrarily changed. By adjusting the feed rate to be lower than the discharge rate of the culture medium, the replacement efficiency of the culture medium within the culture bag can be increased.

In the above aspect, the discharge port of the culture bag may have a diameter larger than a diameter of the feed port. Accordingly, the feed rate of the culture medium toward the culture bag can be set to be lower than the discharge rate from the culture bag, thereby improving the replacement efficiency of the culture medium within the culture bag.

In the above aspect, the cell culturing apparatus may further include a culture-medium preserving means that maintains the culture medium at a temperature suitable for preservation and that feeds the culture medium to the culture-medium retaining means and a liquid-amount sensor that detects an amount of the culture medium within the culture-medium retaining means. When the amount of the culture medium fed from the culture-medium preserving means to the culture-medium retaining means reaches a predetermined amount, the liquid-amount sensor may detect that the amount of the culture medium has reached the predetermined amount and actuate the negative-pressure supplying means. Accordingly, the culture medium can be intermittently fed and discharged at desired intervals with a simple configuration.

In the above aspect, the cell culturing apparatus may further include a culture-medium preserving means that maintains the culture medium at a temperature suitable for preservation and that feeds the culture medium to the culture-medium retaining means and a liquid-amount sensor that detects an amount of the culture medium within the culture-medium retaining means. When the amount of the culture medium fed from the culture-medium preserving means to the culture-medium retaining means reaches a predetermined amount, the liquid-amount sensor may detect that the amount of the culture medium has reached the predetermined amount and actuate the negative-pressure supplying means and the feed-rate adjusting means. Accordingly, the culture medium can be intermittently fed and discharged at desired intervals with a simple configuration.

In the above aspect, the culture medium from the discharge port of the culture bag may drip down through a space within the culture-medium retaining means. Accordingly, the culture medium can be prevented from flowing backward, thereby preventing the interior of the culture bag from being contaminated.

### {Advantageous Effects of Invention}

According to the present invention, since the culture medium in the culture bag can be replaced by using a simple system configuration, the risks of system errors can be reduced, and the risks of adversely affecting the culturing conditions due to errors can be avoided.

### {Brief Description of Drawings}

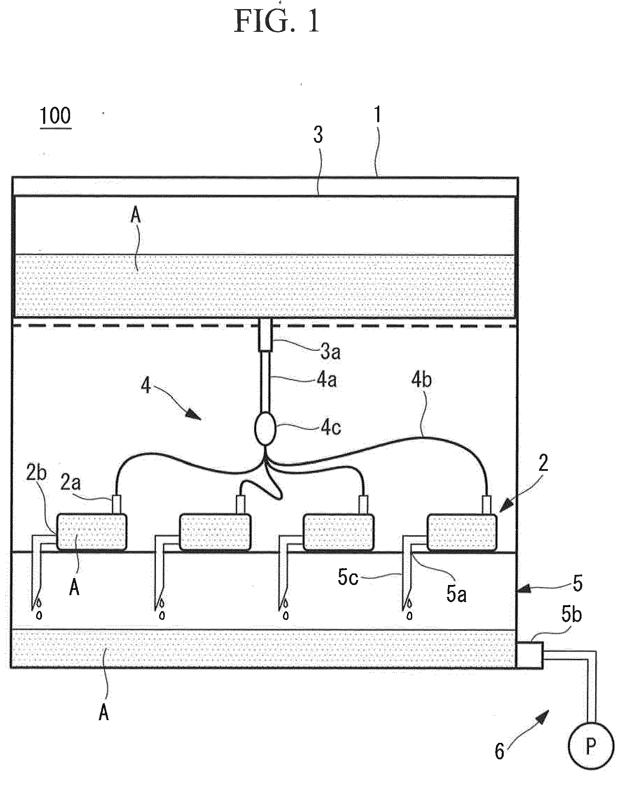

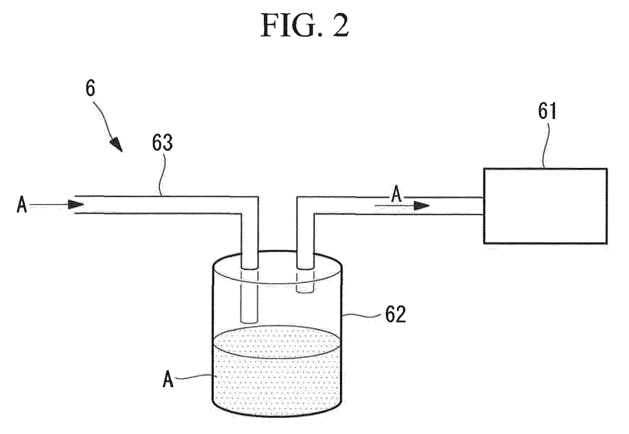

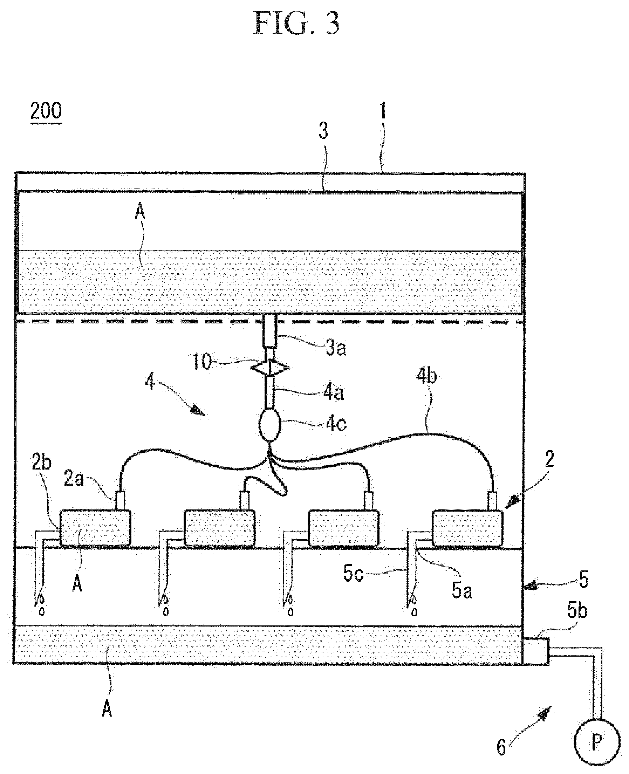

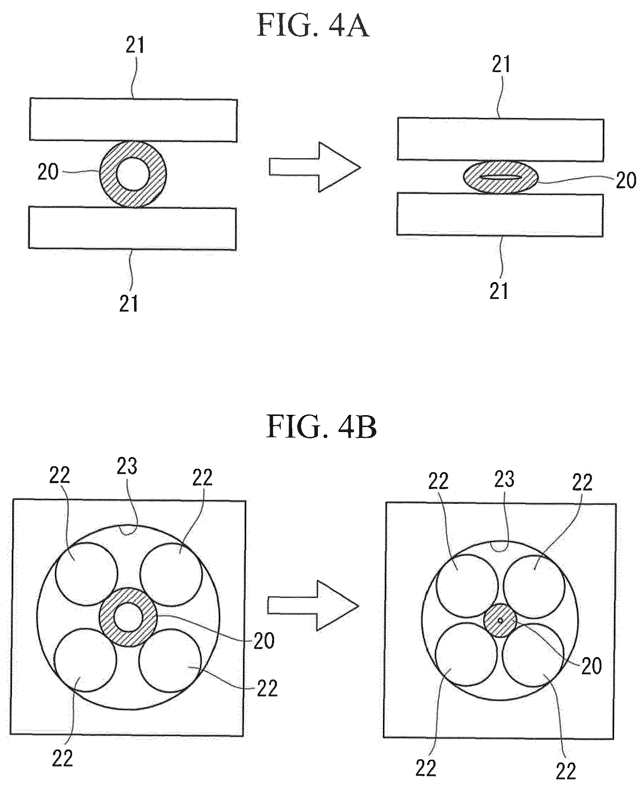

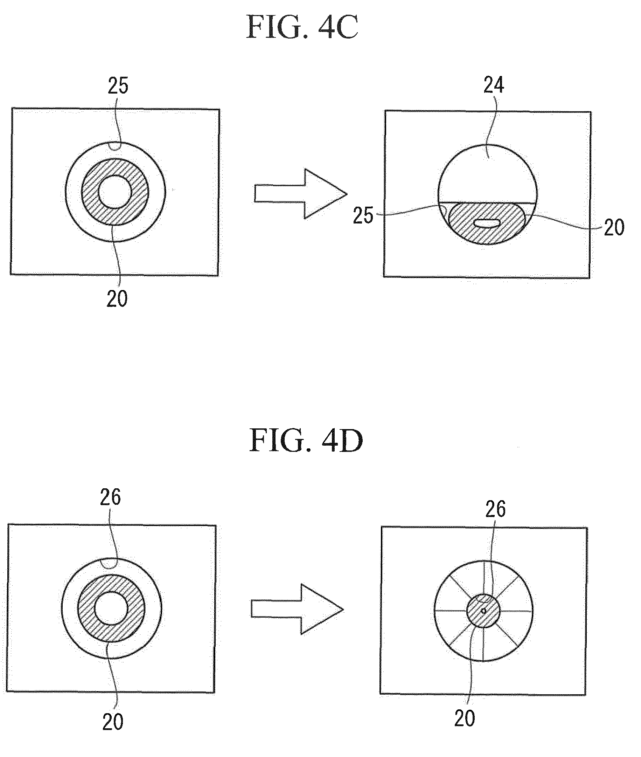

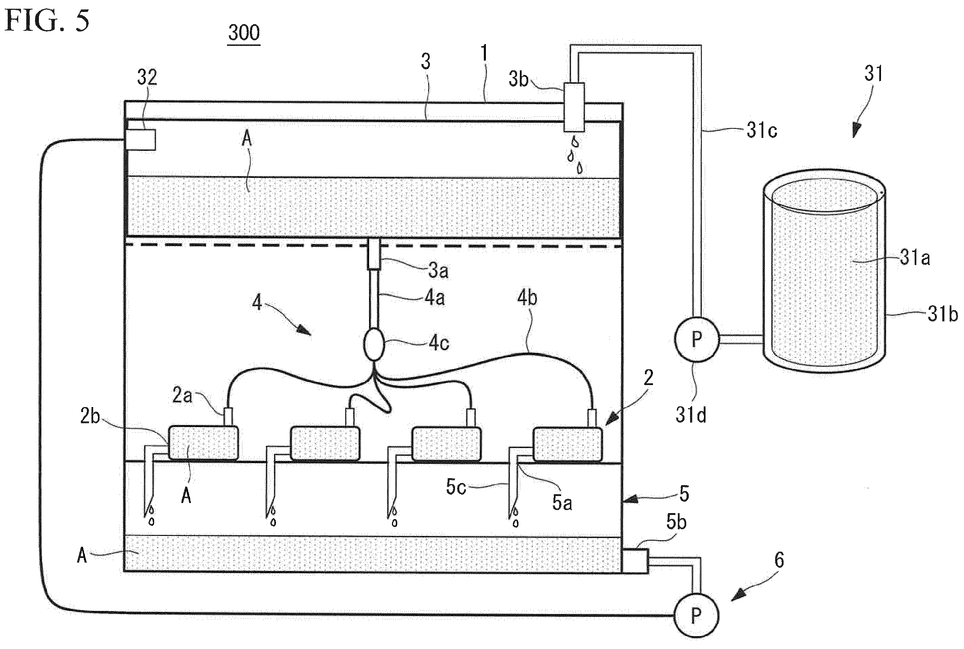

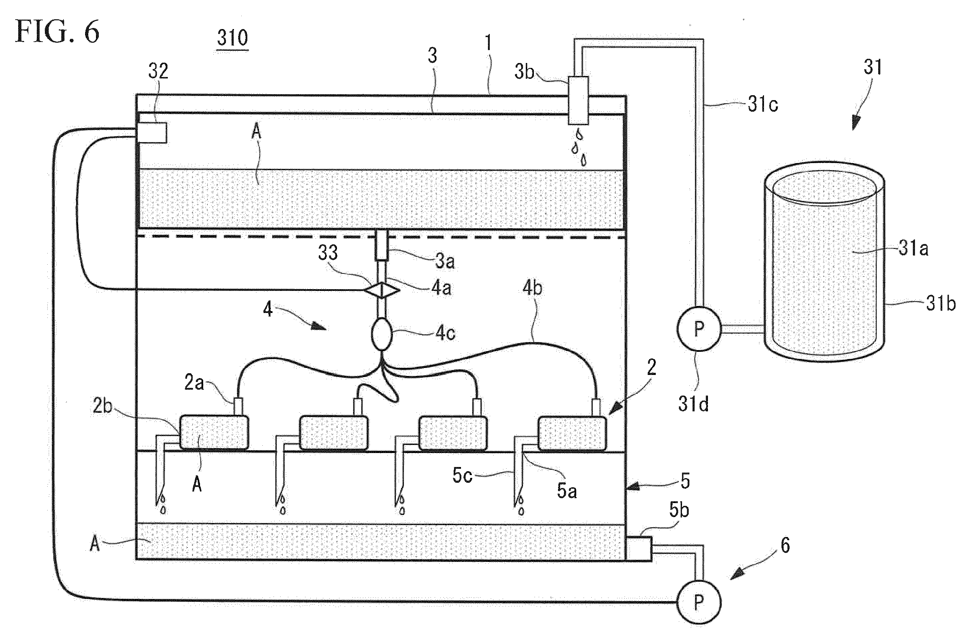

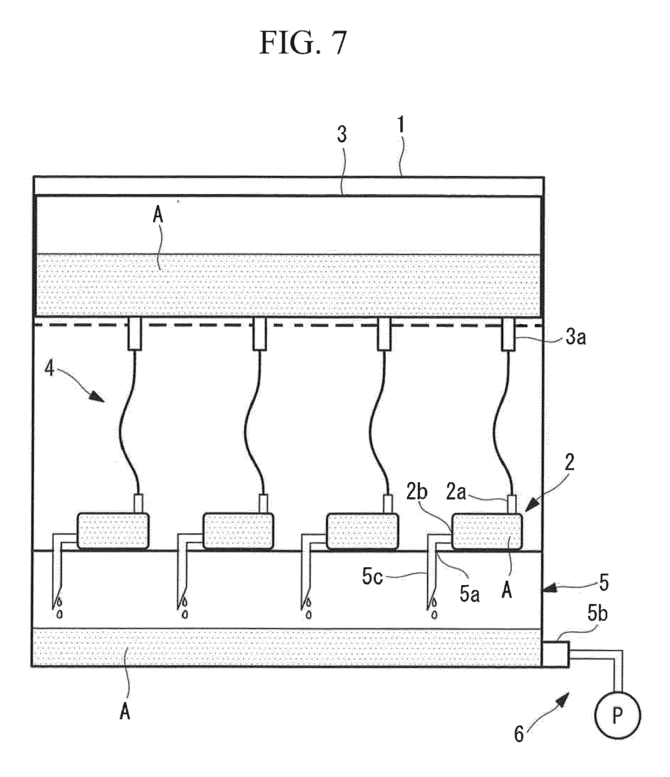

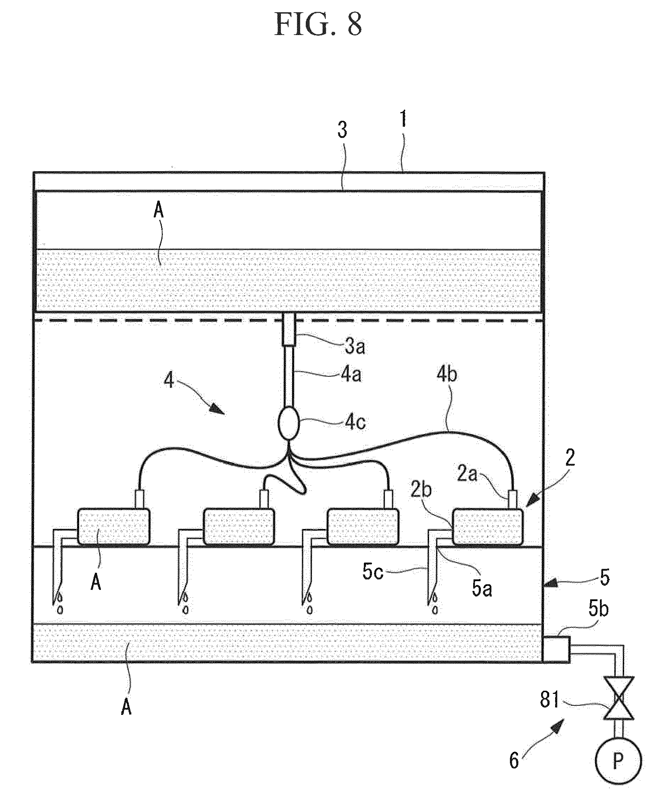

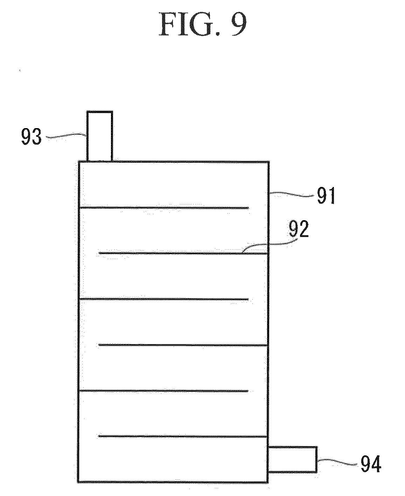

{Fig. 1} Fig. 1 schematically illustrates the configuration of a cell culturing apparatus according to a first embodiment of the present invention.
{Fig. 2} Fig. 2 schematically illustrates the configuration of an example of a negative-pressure supplying means according to the present invention.
{Fig. 3} Fig. 3 schematically illustrates the configuration of a cell culturing apparatus according to a second embodiment of the present invention.
{Fig. 4A} Fig. 4A schematically illustrates the configuration of an example of a feed-rate adjusting means according to the present invention.
{Fig. 4B} Fig. 4B schematically illustrates the configuration of another example of the feed-rate adjusting means according to the present invention.
{Fig. 4C} Fig. 4C schematically illustrates the configuration of another example of the feed-rate adjusting means according to the present invention.
{Fig. 4D} Fig. 4D schematically illustrates the configuration of another example of the feed-rate adjusting means according to the present invention.
{Fig. 5} Fig. 5 schematically illustrates the configuration of a cell culturing apparatus according to a third embodiment of the present invention.
{Fig. 6} Fig. 6 schematically illustrates the configuration of a modification of the cell culturing apparatus according to the third embodiment of the present invention.
{Fig. 7} Fig. 7 schematically illustrates the configuration of a modification of each embodiment of the present invention.
{Fig. 8} Fig. 8 schematically illustrates the configuration of a modification of each embodiment of the present invention.
{Fig. 9} Fig. 9 schematically illustrates the configuration of a culture bag that can be used in the present invention.

### {Description of Embodiments}

### {First Embodiment}

A cell culturing apparatus according to a first embodiment of the present invention will be described below with reference to the drawings.

A cell culturing apparatus 100 according to this embodiment has the configuration shown in Fig. 1 and replaces a culture medium A within a culture bag (cell culture bag) 2 set within an incubator.

The cell culturing apparatus 100 includes the culture bag 2 that accommodates cells and the culture medium A and that cultures cells, a culture-medium retaining means 3, a culture-medium feeding means 4 that feeds the culture medium A from the culture-medium retaining means 3 to the culture bag 2, and culture-medium discharging means 5 and 6 that discharge the culture medium A from the culture bag 2.

The culture-medium retaining means 3 is formed of, for example, a box-shaped container set inside an incubator 1 and is for retaining the culture medium (cell culture solution) A therein. The temperature of the culture medium A is maintained at the temperature inside the incubator 1 (e.g., 37°C).

The culture medium A heated in the culture-medium retaining means 3 to the temperature inside the incubator 1 suitable for cell culturing (e.g., 37°C) is fed to the culture bag 2 via the culture-medium feeding means 4 within the incubator 1. The culture-medium retaining means 3 is provided with a discharge port 3a through which the culture medium A is discharged to the culture-medium feeding means 4. The discharge port 3a is preferably set at a location where the culture medium A therein can be completely discharged therefrom, namely, at the bottom surface or the lower side surface of the culture-medium retaining means 3.

The culture-medium feeding means 4 includes a tubular member, such as a tube, connecting the discharge port 3a of the culture-medium retaining means 3 to a feed port 2a of the culture bag 2. In a case where a plurality of culture bags 2 are used, a tubular member 4a extending from the discharge port 3a of the culture-medium retaining means 3 branches off into a plurality of tubular members 4b at a branch section 4c, and the plurality of tubular members 4b are connected to the feed ports 2a of different culture bags 2.

The culture bag 2 has the feed port 2a connected to the tubular member 4b of the culture-medium feeding means 4 and a discharge port 2b through which the culture medium A is discharged outside the culture bag 2. Although the feed port 2a and the discharge port 2b may be set at freely-chosen positions, it is preferable that the feed port 2a and the discharge port 2b be set at positions as far apart from each other as possible to improve the replacement efficiency of the culture medium.

The diameter (or the cross-sectional area) of the discharge port 2b is preferably larger than the diameter (or the cross-sectional area) of the feed port 2a. This causes the old culture medium A within the culture bag 2 to be discharged faster than the feeding of a new culture medium A, thereby increasing the replacement efficiency of the culture medium A.

The culture bag 2 is a culture bag for adherent cells and is preferably given a surface treatment for facilitating the adhesion of cells.

The culture medium A within the culture bag 2 is discharged from the discharge port 2b by the culture-medium discharging means. The culture-medium discharging means includes a waste retaining means 5 and a negative-pressure supplying means 6.

The waste retaining means 5 is formed of, for example, a box-shaped container disposed below the culture bag 2. The waste retaining means 5 has a waste feed port 5a connected to a tubular member, such as a tube, extending from the discharge port 2b of the culture bag 2 and also has a waste discharge port 5b through which the culture medium A is discharged outside the waste retaining means 5. The waste feed port 5a is set at the upper surface of the waste retaining means 5 so as to be located above the liquid level, in the vertical direction, of the culture medium A retained in the waste retaining means 5. The culture medium A fed through the waste feed port 5a drips down through the space in the waste retaining means 5 and is discharged through the waste discharge port 5b. The culture medium A drips down through the space in the waste retaining means 5 in this manner so that the culture medium A is prevented from flowing backward, thereby preventing the interior of the culture bag 2 from being contaminated. The waste discharge port 5b is preferably provided at the bottom surface or the lower side surface of the waste retaining means 5 so as to be located below the liquid level of the culture medium A.

The waste discharge port 5b is connected to the negative-pressure supplying means 6.

As shown in Fig. 2, the negative-pressure supplying means 6 may include, for example, a means equipped with a pump 61, a waste container 62, and a suction port 63 communicating with the interior of the waste container 62. In this example, negative pressure generated by the pump 61 is transmitted to the suction port 63 via the waste container 62. By connecting the suction port 63 of the negative-pressure supplying means 6 to the waste discharge port 5b of the waste retaining means 5, the interior of the waste retaining means 5 is set in a negative pressure state and the discharge port 2b of the culture bag 2 is set in a negative pressure state, so that the culture medium A can be suctioned from the culture bag 2 toward the waste container 62.

A liquid feed pump, such as a peristaltic pump, may be used as the pump of the negative-pressure supplying means 6. In this case, the liquid feed pump may be set on a tubular member constituting the suction port 63.

Next, an example of a procedure for replacing the culture medium A by using the cell culturing apparatus 100 according to this embodiment will be described.

A user of this apparatus first prepares the culture bag 2 containing the culture medium A and cells (adherent cells) therein and connects the feed port 2a of the culture bag 2 to the tubular member 4b of the culture-medium feeding means 4 within the incubator 1 and also connects the discharge port 2b of the culture bag 2 to the waste feed port 5a of the waste retaining means 5 by means of a tubular member 5c within the incubator 1.

When the culture medium A needs to be replaced, the user first actuates the negative-pressure supplying means 6 so that the interior of the waste retaining means 5 is set in a negative pressure state. Accordingly, the culture medium A within the culture bag 2 is suctioned into the waste retaining means 5 via the tubular member 5c. At the same time, the interior of the culture bag 2 is also set in a negative pressure state, so that the culture medium A within the culture-medium retaining means 3 is fed into the culture bag 2 via the culture-medium feeding means 4.

The user stops the negative-pressure supplying means 6 when an appropriate amount of the culture medium A is discharged from the culture bag 2. Accordingly, the negative pressure in the waste retaining means 5 is released, so that the discharging of the culture medium A from the culture bag 2 stops. When the pressure within the culture bag 2 returns to normal pressure, the feeding of the culture medium A into the culture bag 2 also stops.

In this embodiment, a control unit used for remotely controlling the negative-pressure supplying means 6 may be provided. For example, this allows the user to replace the culture medium A at a desired timing while remotely monitoring it using a monitoring system (not shown).

Furthermore, the control unit may automatically control the negative-pressure supplying means 6 in accordance with a preset program.

### {Second Embodiment}

Next, a cell culturing apparatus according to a second embodiment of the present invention will be described below with reference to the drawings.

A cell culturing apparatus 200 according to this embodiment has the configuration shown in Fig. 3 and differs from that in the first embodiment in being provided with a feed-rate adjusting means 10 in a tubular member of the culture-medium feeding means 4. Other configurations are the same as those in the first embodiment.

The feed-rate adjusting means 10 is disposed on a tubular member (such as a tube) of the culture-medium feeding means 4 and deforms the tubular member by applying an external force thereto in the radial direction so as to reduce the cross-sectional area of the lumen of the tubular member, thereby limiting the flow of a solution and reducing the flow rate thereof. When the external force is released, the tubular member recovers its original state due to an elastic force of the tubular member, so that the flow rate can be increased. Accordingly, the feed-rate adjusting means 10 adjusts the flow rate of the solution flowing through the tubular member by increasing or decreasing the external force applied to the tubular member.

Figs. 4A to 4D illustrate examples of how the external force is applied to the tubular member by the feed-rate adjusting means 10. Fig. 4A illustrates an example in which a tubular member 20 is nipped by two plate-like members 21. Fig. 4B illustrates an example in which the tubular member 20 extending through a through-hole 23 is nipped by a plurality of spherical (or cylindrical) members 22. Fig. 4C illustrates an example in which the tubular member 20 extending through a through-hole 25 is nipped by a shutter-like member 24. Fig. 4D illustrates an example in which the tubular member 20 is deformed by reducing the inner diameter of a through-hole 26 having the tubular member extending therethrough. As an alternative to the above examples, any mechanism that can deform a tubular member by applying an external force thereto may be employed as the feed-rate adjusting means 10.

By using the feed-rate adjusting means 10, the user can adjust the feed rate of the culture medium A toward the culture bag 2 (can also set the culture-medium feed rate to zero to stop feeding the culture medium A). By setting the rate at which the culture medium A is fed to the culture bag 2 to be lower than the rate at which the culture medium A is discharged from the culture bag 2, the efficiency with which the old culture medium A is replaced with the new culture medium A can be increased.

Although the procedure for replacing the culture medium A by using the cell culturing apparatus 200 according to this embodiment is the same as that in the first embodiment, the user can adjust the feed rate of the culture medium A toward the culture bag 2 by using the feed-rate adjusting means 10 in this embodiment.

In this embodiment, a control unit used for remotely controlling the negative-pressure supplying means 6 and the feed-rate adjusting means 10 may be provided. For example, this allows the user to replace the culture medium A at a desired timing while remotely monitoring it using a monitoring system (not shown).

Furthermore, the control unit may automatically control the negative-pressure supplying means 6 and the feed-rate adjusting means 10 in accordance with a preset program.

### {Third Embodiment}

Next, a cell culturing apparatus according to a third embodiment of the present invention will be described below with reference to the drawings.

A cell culturing apparatus 300 according to this embodiment has the configuration shown in Fig. 5 and differs from that in the first embodiment in being provided with a culture-medium preserving means 31 and a liquid-amount sensor 32 provided within the culture-medium retaining means 3. Other configurations are the same as those in the first embodiment.

The culture-medium preserving means 31 includes a culture-medium preserving container 31a and is connected to an opening (feed port 3b) of the culture-medium retaining means 3 by means of a tubular member 31c, such as a tube. A liquid feed pump 31d, such as a peristaltic pump, is provided on the tubular member 31c , such that the culture medium A within the culture-medium preserving container 31a can be fed to the culture-medium retaining means 3 by the liquid feed pump 31d. A temperature adjusting means 31b maintains the culture medium A within the culture-medium preserving container 31a at a temperature suitable for preserving the culture medium (e.g., 4°C). The temperature adjusting means 31b is, for example, a refrigerated container.

Although the feed port 3b of the culture-medium retaining means 3 may be set at a freely-chosen position, it is preferable that the feed port 3b be set at the upper surface of the culture-medium retaining means 3 so that the fed culture medium A drips down through the space in the culture-medium retaining means 3. This prevents the culture medium A from flowing backward, thereby preventing the interior of the culture-medium preserving container 31a from being contaminated.

The liquid-amount sensor 32 detects the amount of culture medium A within the culture-medium retaining means 3. When the amount of culture medium A within the culture-medium retaining means 3 reaches a predetermined amount, the liquid-amount sensor 32 detects this and sends information to the negative-pressure supplying means 6 so as to actuate the negative-pressure supplying means 6. The negative-pressure supplying means 6 may be set to stop when a predetermined time period elapses from the start of actuation. Alternatively, when the amount of culture medium A within the culture-medium retaining means 3 decreases to a predetermined amount, the liquid-amount sensor 32 may detect this and send information to the negative-pressure supplying means 6, and the negative-pressure supplying means 6 may be set to stop in response to the information from the liquid-amount sensor 32. The information from the liquid-amount sensor 32 to the negative-pressure supplying means 6 may be transmitted in either a wired or wireless manner.

An example of the liquid-amount sensor 32 is a sensor that can optically or electrically detect the liquid level of the culture medium A within the culture-medium retaining means 3. Examples include a sensor that detects electricity generated as a result of the culture medium A coming into contact with an electrode set at a predetermined position, a sensor that detects the position of the liquid level on the basis of a change in electrostatic capacitance, a sensor that optically detects the position of the liquid level on the basis of reflected light from the liquid surface, and a sensor that reflects ultrasonic waves or electric waves onto the liquid surface and detects the reflection time period. Alternatively, the liquid-amount sensor 32 may detect the position of a float floating in the culture medium A within the culture-medium retaining means 3 or may detect a weight change (pressure change) of the culture-medium retaining means 3.

Next, a procedure for replacing the culture medium by using the cell culturing apparatus 300 according to this embodiment will be described.

A user of this apparatus first prepares the culture bag 2 containing the culture medium A and cells (adherent cells) therein and connects the feed port 2a of the culture bag 2 to the tubular member 4b of the culture-medium feeding means 4 within the incubator 1 and also connects the discharge port 2b of the culture bag 2 to the waste feed port 5a of the waste retaining means 5 by means of the tubular member 5c within the incubator 1.

The culture-medium preserving means 31 is connected to the feed port 3b of the culture-medium retaining means 3, and the liquid feed pump 31d is actuated so as to start feeding the culture medium A to the culture-medium retaining means 3. The culture medium A fed to the culture-medium retaining means 3 is heated to the temperature inside of the incubator 1 (temperature suitable for cell culturing). When the culture medium A within the culture-medium retaining means 3 reaches a predetermined amount, the liquid-amount sensor 32 detects this and sends information to the negative-pressure supplying means 6 so as to actuate the negative-pressure supplying means 6, whereby the interior of the waste retaining means 5 is set in a negative pressure state. The negative-pressure supplying means 6 is in a stopped state prior to the actuation of the liquid-amount sensor 32. Accordingly, the culture medium A within the culture bag 2 is suctioned into the waste retaining means 5 via the tubular member 5c. At the same time, the interior of the culture bag 2 is also set in a negative pressure state, so that the culture medium A within the culture-medium retaining means 3 is fed into the culture bag 2 via the culture-medium feeding means 4.

The negative-pressure supplying means 6 stops when a predetermined time period elapses from the start of actuation. Accordingly, the negative pressure in the waste retaining means 5 is released, so that the discharging of the culture medium A from the culture bag 2 stops. Furthermore, when the pressure within the culture bag 2 returns to normal pressure, the feeding of the culture medium A into the culture bag 2 also stops, so that the amount of culture medium A within the culture-medium retaining means 3 starts to increase. When the culture medium A within the culture-medium retaining means 3 reaches the predetermined amount, the liquid-amount sensor 32 detects this, and the above-described steps are repeated.

Fig. 6 illustrates a modification of this embodiment.

A cell culturing apparatus 310 according to this modification has the configuration shown in Fig. 6. The cell culturing apparatus 310 further includes a feed-rate adjusting means 33 on a tubular member of the culture-medium feeding means 4 and can adjust the feed rate of the culture medium A toward the culture bag 2 (can also set the feed rate of the culture medium A to zero to stop feeding it) by using the feed-rate adjusting means 33 on the basis of the amount of liquid detected by the liquid-amount sensor 32. For example, the feed-rate adjusting means 33 has a configuration similar to that of the feed-rate adjusting means 10 shown in Figs. 4A to 4D.

According to this modification, when the culture medium A within the culture-medium retaining means 3 reaches a predetermined amount, the liquid-amount sensor 32 detects this and sends information to the negative-pressure supplying means 6 and the feed-rate adjusting means 33 so as to actuate the negative-pressure supplying means 6 and the feed-rate adjusting means 33, whereby the discharging of the culture medium A from the culture bag 2 and the feeding of the culture medium A into the culture bag 2 can be controlled.

Next, an example of how the negative-pressure supplying means 6 and the feed-rate adjusting means 33 are controlled by the liquid-amount sensor 32 will be described.

Prior to actuation of the liquid-amount sensor 32, the negative-pressure supplying means 6 is in a stopped state, and the feed-rate adjusting means 33 is in a state where it is blocking the feeding of the culture medium A (i.e., a state where the feed-rate adjusting means 33 has closed the flow path such that the feed rate of the culture medium A is zero). When the culture medium A within the culture-medium retaining means 3 reaches a predetermined amount, the liquid-amount sensor 32 detects this and sends information to the negative-pressure supplying means 6 so as to actuate the negative-pressure supplying means 6, whereby the interior of the waste retaining means 5 is set in a negative pressure state. Accordingly, the culture medium A within the culture bag 2 is suctioned into the waste retaining means 5 via the tubular member 5c.

When a predetermined time period elapses after sending the information to the negative-pressure supplying means 6, the liquid-amount sensor 32 sends the information to the feed-rate adjusting means 33 so that the flow path is opened, whereby feeding of the culture medium A commences. The negative-pressure supplying means 6 and the feed-rate adjusting means 33 are set to stop when a predetermined time period elapses after receiving the information from the liquid-amount sensor 32. When the negative-pressure supplying means 6 and the feed-rate adjusting means 33 stop, the amount of culture medium A within the culture-medium retaining means 3 starts to increase. When the culture medium A within the culture-medium retaining means 3 reaches the predetermined amount, the liquid-amount sensor 32 detects this, and the above-described steps are repeated.

By varying the actuation timings of the negative-pressure supplying means 6 and the feed-rate adjusting means 33 in this manner, the replacement efficiency of the culture medium A can be improved.

As an alternative to the liquid-amount sensor 32 sending information to the negative-pressure supplying means 6 and the feed-rate adjusting means 33 with a certain time lag, the liquid-amount sensor 32 may send the information simultaneously to the negative-pressure supplying means 6 and the feed-rate adjusting means 33, and the feed-rate adjusting means 33 may be actuated when a predetermined time period elapses after receiving the information.

It is preferable that the liquid-amount sensor 32, the negative-pressure supplying means 6, and the feed-rate adjusting means 33 each include a timer.

As an alternative to the negative-pressure supplying means 6 and the feed-rate adjusting means 33 stopping when a predetermined time period elapses from the start of actuation, when the amount of culture medium A within the culture-medium retaining means 3 decreases to a predetermined amount, the liquid-amount sensor 32 may detect this and send information to the negative-pressure supplying means 6 and the feed-rate adjusting means 33 so as to stop the negative-pressure supplying means 6 and the feed-rate adjusting means 33.

In this embodiment and the modifications thereof, a control unit used for remotely controlling the liquid feed pump 31d may be provided. For example, this allows the user to replace the culture medium A at a desired timing while remotely monitoring it using a monitoring system (not shown).

Furthermore, the control unit may automatically control the liquid feed pump 31d in accordance with a preset program.

The information from the liquid-amount sensor 32 to the negative-pressure supplying means 6 and the feed-rate adjusting means 33 may be transmitted in either a wired or wireless manner.

According to this embodiment and the modifications thereof, the feed rate of the culture medium A from the culture-medium preserving means 31 toward the culture-medium retaining means 3 is adjusted so that the culture medium A within the culture bag 2 can be replaced at desired intervals.

In each of the above-described embodiments, the culture-medium discharging means 5 and 6 may be set either inside or outside the incubator 1.

Furthermore, in a case where the negative-pressure supplying means 6 includes the waste container 62, the waste retaining means 5 need be not employed, and the waste container 62 may have the function of the waste retaining means 5. In this case, it is preferable that the culture medium A fed into the waste container 62 drips down through the space in the waste container 62. This prevents the culture medium A from flowing backward, thereby preventing the interior of the culture bag 2 from being contaminated.

In a case where a plurality of culture bags 2 are used in each of the above-described embodiments, the tubular member 4a extending from the discharge port 3a of the culture-medium retaining means 3 branches off into the plurality of tubular members 4b at the branch section 4c, and the plurality of tubular members 4b are connected to the feed ports 2a of different culture bags 2. Alternatively, for example, as shown in Fig. 7, the culture-medium retaining means 3 may be provided with a plurality of discharge ports 3a, and the plurality of discharge ports 3a may be individually connected to the feed ports 2a of different culture bags 2 by means of tubular members.

Furthermore, only one culture bag 2 may be provided.

In each of the above-described embodiments, the negative-pressure supplying means 6 may actuate or stop the pump 61 so as to control the supplying of negative pressure to the discharge port 2b of the culture bag 2. Alternatively, as shown in Fig. 8, a flow-path on-off gate 81 may be set on a tubular member that connects the waste retaining means 5 and the negative-pressure supplying means 6, and the supplying of negative pressure may be controlled by opening or closing the flow path of the tubular member in a state where the pump 61 is actuated.

For example, the flow-path on-off gate 81 may be similar to the feed-rate adjusting means 10 and 33 and may be of the types shown in Figs. 4A to 4D.

The flow-path on-off gate 81 may be remotely controlled by a control unit. The remote control may be performed in a wireless or wired manner.

The control unit according to the present invention may be, for example, a personal computer (PC). For example, the PC may have a CPU and a memory and may realize the function of the control unit by causing the CPU to execute a control program stored in the memory.

An example of a culture bag that can be used in the present invention is, for example, a culture bag 91 shown in Fig. 9, which has a flow path therein. By forming a flow path in this manner, the replacement efficiency of the culture medium A can be increased. The flow path may be formed by partially bonding opposing faces of the culture bag and forming boundaries 92 in the space within the culture bag 91. By setting a feed port 93 and a discharge port 94 serving as openings of the culture bag 91 at positions distant from each other in the flow path, the replacement efficiency of the culture medium A can be improved. The diameter (or the cross-sectional area) of the discharge port 94 may be larger than the diameter (or the cross-sectional area) of the feed port 93.

In the present invention, the feed-rate adjusting means 10 and 33 may be liquid feed pumps, such as peristaltic pumps.

### {Reference Signs List}

- 1: incubator
- 2, 91: culture bag
- 2a, 93: feed port
- 2b, 94: discharge port
- 3: culture-medium retaining means
- 4: culture-medium feeding means
- 5: waste retaining means
- 6: negative-pressure supplying means
- 10, 33: feed-rate adjusting means
- 31: culture-medium preserving means
- 32: liquid-amount sensor
- 100, 200, 300: cell culturing apparatus

## Claims

1. A cell culturing apparatus comprising:
a culture-medium retaining means that retains a culture medium for culturing a cell;
a culture bag having a feed port to which the culture medium is fed and a discharge port through which the culture medium is discharged from inside the culture bag;
a culture-medium feeding means that connects the culture-medium retaining means and the feed port of the culture bag and that feeds the culture medium discharged from the culture-medium retaining means to the culture bag;
a negative-pressure supplying means that supplies negative pressure to the discharge port of the culture bag; and
a waste retaining means that retains the culture medium from the discharge port of the culture bag.

2. The cell culturing apparatus according to Claim 1,
wherein the culture-medium feeding means includes a feed-rate adjusting means that adjusts a rate at which the culture medium is fed to the culture bag.

3. The cell culturing apparatus according to Claim 1, further comprising:
a culture-medium preserving means that maintains the culture medium at a temperature suitable for preservation and that feeds the culture medium to the culture-medium retaining means; and
a liquid-amount sensor that detects an amount of the culture medium within the culture-medium retaining means,
wherein, when the amount of the culture medium fed from the culture-medium preserving means to the culture-medium retaining means reaches a predetermined amount, the liquid-amount sensor detects that the amount of the culture medium has reached the predetermined amount and actuates the negative-pressure supplying means.

4. The cell culturing apparatus according to Claim 2, further comprising:
a culture-medium preserving means that maintains the culture medium at a temperature suitable for preservation and that feeds the culture medium to the culture-medium retaining means; and
a liquid-amount sensor that detects an amount of the culture medium within the culture-medium retaining means,
wherein, when the amount of the culture medium fed from the culture-medium preserving means to the culture-medium retaining means reaches a predetermined amount, the liquid-amount sensor detects that the amount of the culture medium has reached the predetermined amount and actuates the negative-pressure supplying means and the feed-rate adjusting means.

5. The cell culturing apparatus according to any one of Claims 1 to 4,
wherein the discharge port of the culture bag has a diameter larger than a diameter of the feed port.

6. The cell culturing apparatus according to any one of Claims 1 to 5,
wherein the culture medium from the discharge port of the culture bag drips down through a space within the culture-medium retaining means.
